# EUROPEAN PATENT APPLICATION

(11) **EP 0 723 787 A2**
(43) Date of publication of application: **31.07.1996**
(21) Application number: 96200161.6
(22) Date of filing: 23.01.1996
(51) Int. Cl.: A61M 39/06

(54) **Haemostatic device**

(30) Priority: 25.01.1995 NL 9500142
(71) Applicant: CORDIS EUROPA N.V., NL-9301 LJ Roden (NL)
(72) Inventor: Bosma, Gjalt, NL-9201 EK Drachten (NL); Jansen, Robert W., NL-9302 BE Roden (NL)
(74) Representative: 't Jong, Bastiaan Jacobus

(57) **Abstract**

The invention relates to a haemostatic device (1) comprising a housing (2) containing a continuous channel (3), and connected to the channel (3) a chamber (10) inside of which an elastic compressible sealing element (11) has been received. Inside the housing (2) an axially displaceable pressure element (12,14), working on the sealing element (11), has been arranged. The sealing element (11) comprises, in line with the channel (3), a depression (16) of which the wall diverges in opposite axial directions from a central section (17).

## Description

The invention relates to a haemostatic device. Such a device is for instance arranged at the proximal end of an introduction member introduced into the blood-stream of a patient. Via the haemostatic device a catheter, a guide wire or similar means can be introduced into the bloodstream. By using the pressure element to apply a force on the sealing element, the latter can be compressed as a result of which it seals against the catheter or similar means.

With the haemostatic device according to the invention a very good seal can be achieved. Because of the shape of the depression a good seal is obtained using a relatively low pressure. Nevertheless, the catheter or similar means experience relatively little friction, so that they can still be moved to and fro.

According to a further development, the measure as set out in claim 2 is employed. In the starting position the sealing element closes off the opening, even when the pressure element applies little or no pressure. When changing the catheter or similar means, the seal remains intact so as to prevent the leaking out of blood. By tightening the pressure member a very good seal is achieved at a high pressure, so that also when injecting for instance contrast medium under high pressure, a reliable seal is maintained.

A very suitable further development is characterised in claim 3. The star-shaped cut allows the passing of a catheter or similar means without having to apply a significant pressure, whilst the seal remains as good as intact. However, as a result of the action of the pressure element, the seal can, also in this situation, be reinforced, and the catheter or similar means can be wedged more or less in the opening of the end wall.

A further development is characterised in claim 4. When introducing a catheter or similar means it will remain located exactly in the centre of the star-shaped cut. The catheter can not move into one of the radii of the star-shaped cut, as a result of which a good combined effect for the purpose of sealing is retained.

In a suitable manner the measure as set out in claim 5 is employed.

The invention will be explained in greater detail in the following description with reference to the attached drawings of an example of an embodiment.
- Figure 1: shows a partly cut through, perspective view of the device according to the invention.
- Figure 2: shows a cross-section along the line II-II of figure 1 at the sealing element.
- Figure 3: shows a cross-section corresponding to figure 2 with a catheter inserted through the sealing element.

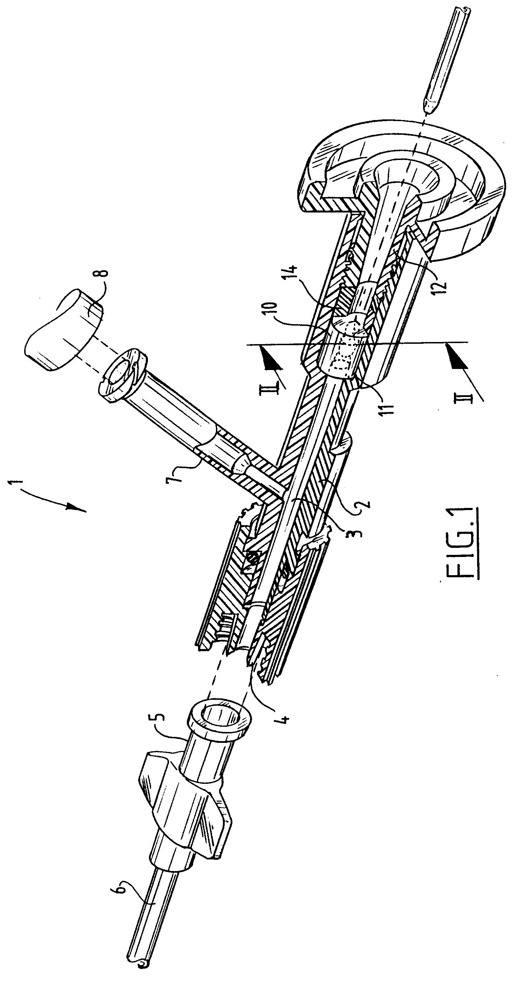

The device 1 of figure 1 comprises a housing 2 with a continuous channel 3. At the left end-section of the housing 2, as seen in figure 1, a male, rotatory Luerlock connector 4 has been arranged with which the device 1 can be connected with the connecting member 5 of a catheter 6.

The housing 2 has also been fitted with a side connection 7 to which an element 8 can be connected, for instance for conveying fluid in the channel 3.

Close to the right end-section of the housing 2, the channel 3 is provided with a dilated section forming a chamber 10. In this case a cylindrical sealing element 11, which has been made of an elastic compressible material, has been received inside this chamber 10. At the right end-section of the housing 2 an internal thread has been arranged adjacent to and coaxial with the chamber 10, in which a pressing screw 12 engages. This pressing screw 12 works via a pressure ring 14 on the sealing element 11. Inside the sealing element 11 a depression 16, of which the wall diverges in opposite axial directions from a central section 17, has been arranged in line with the channel 3.

When the pressing screw 12 is screwed in, pressure is applied to the sealing element 11 via the pressure ring 14, as a result of which the sealing element 11 is compressed. At the central section 17 of the depression 16, it is in particular this depression which is being compressed.

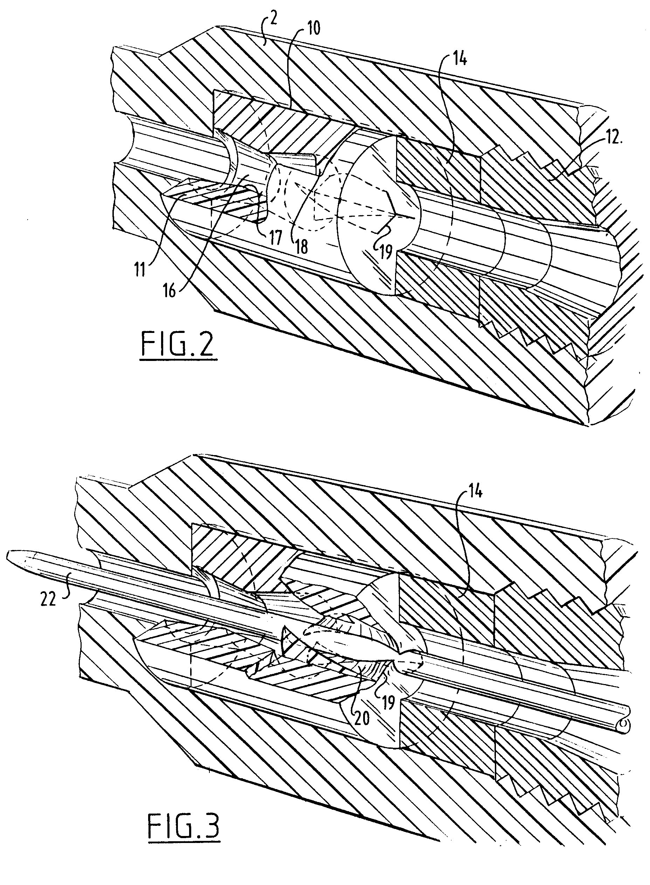

The sealing element 11 has an end wall 18 in which a star-shaped cut 19 with three radii has been arranged. As can be seen clearly in figures 2 and 3, the star-shaped cut 19 has been twisted in a helical shape in axial direction. As a result, the cut surfaces 20 form curved surfaces which will not allow for instance a catheter 22, inserted through the end wall 18, to enter. Consequently the catheter remains accurately positioned in the centre of the cut 19.

The star-shaped cut 19 allows a catheter 22, introduced via the channel section in the pressing screw 12, to pass smoothly. A complete seal remains intact when the pressure is not too high. When the pressure is higher, the pressing screw 12 can be tightened in order to reinforce the seal.

## Claims

1. Haemostatic device comprising a housing containing a continuous channel, and connected to the channel a chamber inside of which an elastic compressible sealing element has been received and wherein, inside the housing, an axially displaceable pressure element working on the sealing element has been arranged, wherein the sealing element comprises, in line with the channel, a depression of which the wall diverges in opposite axial directions from a central section.

2. Haemostatic device as claimed in claim 1, wherein the sealing element has a substantially closed end wall.

3. Haemostatic device as claimed in claim 2, wherein a star-shaped cut has been made in axial direction in the end wall.

4. Haemostatic device as claimed in claim 3, wherein the star-shaped cut has been twisted in a helical shape in axial direction.

5. Haemostatic device as claimed in claim 3 or 4, wherein the star-shaped cut comprises three radii.

6. Sealing element for a haemostatic device as claimed in one of the previous claims.
